# EUROPÄISCHE PATENTSCHRIFT

(11) **EP 1 140 806 B1**
(45) Veröffentlichungstag und Bekanntmachung des Hinweises auf die Patenterteilung: **28.05.2003**
(21) Anmeldenummer: 00901065.3
(22) Anmeldetag: 05.01.2000
(51) Int. Cl.: C07C 281/18, C07C 277/08

(54) **VERFAHREN ZUR HERSTELLUNG VON ROBENIDIN BZW. DESSEN DERIVATE**
METHOD FOR PRODUCING ROBENIDINE OR DERIVATIVES THEREOF
PROCEDE DE PRODUCTION DE ROBENIDINE ET DE SES DERIVES

(30) Priorität: 06.01.1999 EP 99100097; 29.07.1999 US 146107 P
(43) Veröffentlichungstag der Anmeldung: 10.10.2001
(73) Patentinhaber: Lonza AG, 4052 Basel (CH)
(72) Erfinder: HANSELMANN, Paul, CH-3902 Brig-Glis (CH); HILDEBRAND, Stefan, CH-3930 Visp (CH)
(74) Vertreter: Ritthaler, Wolfgang, Dr.rer.nat.Dipl.-Chem
(86) Internationale Anmeldenummer: EP0000050
(87) Internationale Veröffentlichungsnummer: WO00040549

(56) Entgegenhaltungen:
- GB-A- 1 256 723
- US-A- 4 575 560
- CHEMICAL ABSTRACTS, vol. 95, no. 1, 6. Juli 1981 (1981-07-06) Columbus, Ohio, US; abstract no. 6756j, SHESTAKOV ET AL: "Synthesis of khimkoktsid, 1,3-bis(p-chlorobenzylidenamino)guanidine, labeled with the radionuclides carbon-14 and hydrogen-3, and its pharmacokinetics" Seite 6765; Spalte 1; XP002135798 & SHESTAKOV ET AL: KHIM.-FARM. ZH., Bd. 15, Nr. 1, 1981, Seiten 23-28,

## Beschreibung

Die Erfindung betrifft ein neues Verfahren zur Herstellung von Robenidin und dessen Derivaten der allgemeinen Formel 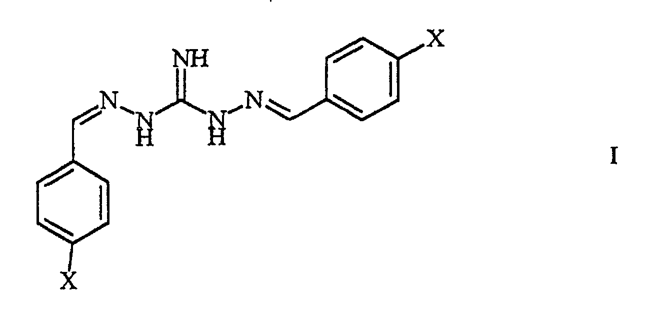 worin X ein Halogenatom bedeutet, ausgehend von Hydrazinhydrat und einem Halogencyan.

Robenidin, 1,3-Bis[4-chlorbenzyliden)-amino]-guanidin, bzw. Robenidinderivate werden zur Bekämpfung von Coccidioseinfektionen in Geflügel und als aktive Antimalaria-Mittel für Warmblüter eingesetzt (DE-A-1 933 112).

Shestakov et al, Khim.-Farm. ZH, Bd. 15, Nr. 1, 1981, Seiten 23-28, offenbart die Herstellung von Robenidin, das ¹⁴C und ³H als Isotope enthält. Ether und i-PrOH werden als Lösungmittel eingesetzt.

Ein Verfahren zur Herstellung von Robenidin ist ebenfalls aus der DE-A-1 933 112 bekannt. Bei diesem Verfahren wird Dichlorbenzaldehyd oder p-Chlorbenzaldehyd mit 1,3-Diaminoguanidinnitrat in Ethanol zum gewünschten Produkt umgesetzt. Nachteilig bei diesem Verfahren ist, dass das Produkt nur in mässiger Ausbeute erhalten wird.

Aufgabe der vorliegenden Erfindung war es, ein "Eintopfverfahren" zur Herstellung von Robenidin zur Verfügung zu stellen, in welchem das gewünschte Produkt in hervorragender Ausbeute und Reinheit erhalten wird.

Diese Aufgabe wird mit dem Verfahren gemäss Anspruch 1 gelöst.

Erfindungsgemäss wird das Verfahren derart durchgeführt, dass man in einer ersten Stufe Hydrazinhydrat mit einer Cyanverbindung der allgemeinen Formel

Y-CN II

worin Y ein Halogenatom oder Tosyl bedeutet, in einem C₁₋₄-Alkohol, oder einem C₁₋₄-Alkohol-Wasser-Gemisch als Lösungsmittel zu einem Diaminoguanidin der allgemeinen Formel 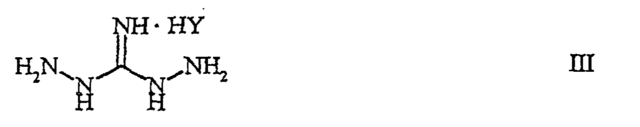 worin Y die genannte Bedeutung hat, umsetzt und dieses, ohne Isolierung, direkt in einer zweiten Stufe mit einem p-Halogenbenzaldehyd der allgemeinen Formel 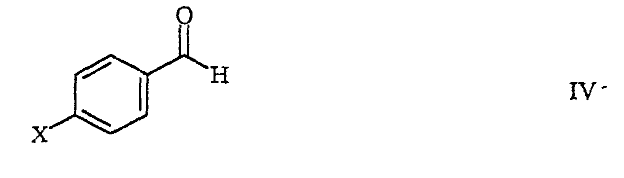 worin X ein Halogenatom bedeutet, in einem C₁₋₄-Alkohol-Wasser-Gemisch als Lösungsmittel in das Endprodukt der Formel 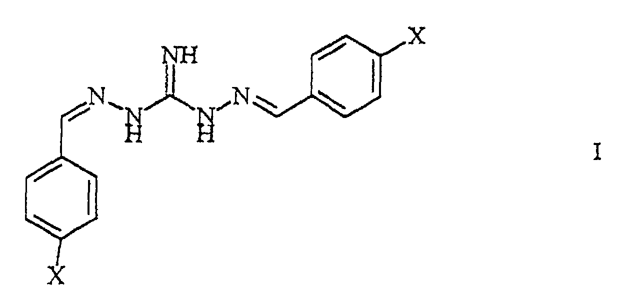 worin X die genannte Bedeutung hat, oder eines seiner Salze, überführt.

Y bedeutet Halogen, beispielsweise Fluor, Chlor, Brom oder Iod, oder Tosyl, bevorzugt Chlor, Brom oder Tosyl. Dementsprechend werden als Cyanverbindungen in der ersten Stufe bevorzugt Chlor- oder Bromcyan oder Tosylcyanid eingesetzt. Besonders bevorzugt wird Chlorcyan eingesetzt.

Als C₁₋₄-Alkohole können Methanol, Ethanol, Propanol, Isopropanol, Butanol, Isobutanol oder tert-Butanol eingesetzt werden. Vorzugsweise wird Isopropanol eingesetzt.

Die erste Stufe wird in einem C₁₋₄-Alkohol oder in einem C₁₋₄-Alkohol-Wasser-Gemisch, insbesondere in einem C₁₋₄-Alkohol-Wasser-Gemisch durchgeführt. Dabei wird zweckmässig ein C₁₋₄-Alkohol-Wasser-Mischungsverhältnis (Gew.-%) von 1:1 bis 15:1, vorzugsweise von 4:1 bis 10:1, eingestellt.

Die Edukte, das Hydrazinhydrat und die Cyanverbindungen wie Chlorcyan, sind käufliche Verbindungen.

Die Umsetzung der ersten Stufe wird zweckmässig bei einer Temperatur von -30 bis 50 °C, vorzugsweise bei einer Temperatur von -10 bis 30 °C, insbesondere bei einer Temperatur von 0 bis 20 °C, durchgeführt.

X bedeutet ein Halogenatom wie Fluor, Chlor, Brom oder Iod. Vorzugsweise hat X die Bedeutung von Chlor oder Brom, besonders bevorzugt von Chlor. Als p-Halogenbenzaldehyde können dementsprechend beispielsweise p-Chlorbenzaldehyd oder p-Brombenzaldehyd eingesetzt werden. Vorzugsweise wird p-Chlorbenzaldehyd eingesetzt.

Zweckmässig wird das Verfahren in der zweiten Stufe bei einer Temperatur von 50 bis 90 °C, vorzugsweise von 65 bis 85 °C, durchgeführt.

Die zweite Stufe wird in einem C₁₋₄-Alkohol-Wasser-Gemisch durchgeführt.

Dabei wird zweckmäßig ein Verhältnis Wasser/C₁₋₁₄-Alkohol (Gew.-%) von 10:1 bis 1:1, vorzugsweise von 2:1 bis 1:1, eingestellt.

In einer besonders bevorzugten Ausführungsform wird das in der ersten Stufe verwendete Lösungsmittel vor der zweiten Stufe mit Wasser verdünnt, insbesondere wenn in der ersten Stufe kein wässriges Lösungsmittel verwendet wurde.

Zweckmässig wird die Umsetzung in der zweiten Stufe bei einem pH-Wert geringer als 3, vorzugsweise bei einem pH-Wert von 0,5 bis 1,5 durchgeführt. Dabei wird zweckmässig der pH-Wert durch Zugabe einer Mineralsäure wie beispielsweise Salzsäure, Salpetersäure oder Schwefelsäure eingestellt.

Nach einer üblichen Umsetzungszeit von 0,5 bis 3 h kann dann das Robenidin bzw. ein Derivat davon durch übliche Aufarbeitungsmethoden als Salz in hervorragender Ausbeute und Reinheit erhalten werden. Erfindungsgemäß herstellbare Salze des Robenidins bzw. der Robenidinderivate sind beispielsweise die Hydrohalogenidsalze, beispielsweise die Hydrochloride oder Hydrobromide.

### Beispiele

### Herstellung von Robenidin

### Beispiel 1

1.1 Zu einer auf 0 °C abgekühlten Lösung von 25,0 g (0,50 mol) Hydrazinhydrat in 200 ml Isopropanol wurden innerhalb 40 Minuten 16,0 g (0,26 mol) Chlorcyan so eingeleitet, dass die Temperatur 12 °C nicht überstieg. Zu diesem Zeitpunkt wurde der pH gemessen und ein Wert von 9,9 erhalten. Danach wurde solange portionsweise je 1,0 g Chlorcyan zugegeben, bis der pH <4 war. Dies war nach der Zugabe von 3×1,0 g Chlorcyan der Fall (pH = 3,8). Nach weiteren 20 Minuten Rühren bei 10 °C wurden 130 ml H₂O zugegeben und der pH wurde tropfenweise mit 10,63 g HCl (konz.) auf 1,0 gestellt. Das Reaktionsgemisch wurde anschliessend auf 70 °C aufgeheizt. Bei einer Innentemperatur von 61 °C wurde mit der Zudosierung von 61,55 g (0,44 mol) geschmolzenem p-Chlorbenzaldehyd (Smp.: 46 °C) begonnen. Dieses Zutropfen dauerte 20 Minuten. Die weisse Suspension wurde innerhalb 10 Minuten auf 80 °C aufgeheizt und dann während weiterer 45 Minuten bei 80 °C gerührt. Das Reaktionsgemisch wurde anschliessend auf 15 °C abgekühlt und zentrifugiert. Der Rückstand wurde solange portionsweise mit je 50 ml H₂O gewaschen bis das Waschwasser einen pH >3 aufwies. Dies war nach 6×50 ml H₂O der Fall. Weitere Waschungen mit Isopropanol (2×50 ml) und Ethylacetat (2×50 ml) lieferten 81,70 g feuchtes Robenidin-Hydrochlorid, das im Trockenschrank bei 90 °C/20 mbar während 48 h getrocknet wurde. Es wurden 75,70 g (82 %) Robenidin-Hydrochlorid als weisser Feststoff mit einem Gehalt von 99,1 % (HPLC) erhalten.
1.2 Die Umsetzung wurde analog zu Beispiel 1.1 durchgeführt, jedoch wurde eine exakte Menge Chlorcyan (0,26 ml) zugegeben, ohne darauf zu achten ob sich ein pH von kleiner als 4 einstellt. Dabei wurde Robenidin-Hydrochlorid mit gleicher Ausbeute isoliert.

### Beispiel 2

Zu einer auf 0°C abgekühlten Lösung von 25,0 g (0,50 mol) Hydrazinhydrat in 215 ml Isopropanol/Wasser 88:12 (Gew.-%) wurden innerhalb von 3 Stunden 15,5 g (0,25 mol) Chlorcyan so eingeleitet, dass die Temperatur 20 °C nicht überstieg. Nach einer Nachreaktionszeit von 10 Minuten bei 10 °C wurden 155 ml H₂O zugegeben und der pH wurde mit 16,2 g konz. HCl auf 1,0 gestellt. Die nun klare Lösung wurde anschliessend auf 70 °C geheizt und 61,5 g (0,44 mol) geschmolzenes p-Chlorbenzaldehyd wurden zugetropft. Danach wurde die weisse Suspension auf 80 °C geheizt und während weiteren 45 Minuten bei dieser Temperatur gerührt. Das Reaktionsgemisch wurde auf 15 °C abgekühlt und zentrifugiert. Waschen des Rückstandes mit H₂O (6×50 ml) und Isopropanol (2×50 ml) und Trocknen (im Trockenschrank bei 90 °C/20 mbar während 15 Stunden) lieferten 77,5 g (84 %; bzgl. Hydrazinhydrat) Robenidin-Hydrochlorid als weissen Feststoff mit einem Gehalt von 99,6 % (HPLC).

### Beispiele 3 bis 8

Die Beispiele 3 bis 8 wurden unter an sich gleichen Bedingungen wie in Beispiel 1 oder 2 durchgeführt, jedoch unter Variation des Lösungsmittels. Die Ergebnisse der Beispiele 1 bis 8 sind in Tabelle 1 zusammengefasst.

**Tabelle 1**

| **Beispiel** | **Lösungsmittel der 1. Stufe** | **Lösungsmittel der 2. Stufe**^{**1)**} | **Ausbeute [%]** | **Gehalt**^{**2)**} **[%]** |
|---|---|---|---|---|
| 1 | Isopropanol (i-PrOH) | i-PrOH/H₂O - Gemisch | 82 | 99,1 |
| 2 | i-PrOH/H₂O 88:12 | i-PrOH/H₂O - Gemisch | 84 | 99,6 |
| 3 | Methanol (MeOH) | MeOH/H₂O - Gemisch | 73 | 89,0 |
| 4 | Ethanol (EtOH)/H₂O 96:4 | EtOH/H₂O - Gemisch | 77 | 92,7 |
| 5 | n-Butanol | n-BuOH/H₂O - Gemisch | 76 | 92,2 |
| 6 | 2-Butanol | 2-Butanol/H₂O - Gemisch | 69 | 89,6 |
| 7 | MeOH | MeOH/i-PrOH/H₂O 4:3:1 | 76 | 83,2 |
| 8 | MeOH | MeOH | 64 | 85,1 |

| | | | | |
|---|---|---|---|---|
| 1) Unter dem Lösungsmittel der 2. Stufe wird das Lösungsmittelgemisch verstanden, das nach der Chlorcyan- aber vor der Chlorbenzaldehyd-Zudosierung durch Zugabe von Wasser (ausser Beispiel 8, in welchem mit Methanol verdünnt wurde) eingestellt wird. | | | | |
| 2) Mittels HPLC ermittelt. | | | | |

## Patentansprüche

1. Verfahren zur Herstellung von Robenidin und dessen Derivaten der allgemeinen Formel 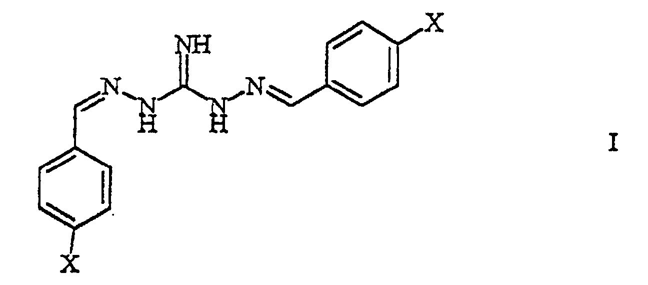 worin X ein Halogenatom bedeutet, sowie Salzen davon, **dadurch gekennzeichnet, dass** in einer ersten Stufe Hydrazinhydrat mit einer Cyanverbindung der allgemeinen Formel
Y-CN II
worin Y ein Halogenatom oder Tosyl bedeutet, in einem C₁₋₄-Alkohol oder einem C₁₋₄-Alkohol-Wasser-Gemisch als Lösungsmittel zu einem Diaminoguanidin der allgemeinen Formel 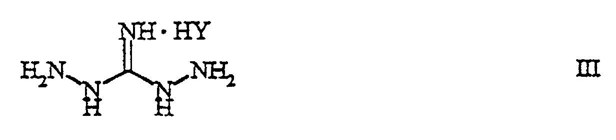 worin Y die genannte Bedeutung hat, umgesetzt wird und dieses, ohne Isolierung, direkt in einer zweiten Stufe mit einem p-Halogenbenzaldehyd der allgemeinen Formel 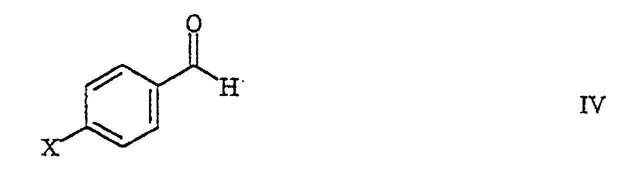 worin X die genannte Bedeutung hat, in einem C₁₋₄-Alkohol-Wasser-Gemisch als Lösungsmittel in das Endprodukt der Formel I überführt wird.

2. Verfahren nach Anspruch 1, **dadurch gekennzeichnet, dass** die Umsetzung in der ersten Stufe bei einer Temperatur von -30 bis 50 °C durchgeführt wird.

3. Verfahren nach einem der Ansprüche 1 oder 2, **dadurch gekennzeichnet, dass** als Cyanverbindung Chlorcyan eingesetzt wird.

4. Verfahren nach einem der Ansprüche 1 bis 3, **dadurch gekennzeichnet, dass** in der zweiten Stufe als p-Halogenbenzaldehyd p-Chlorbenzaldehyd eingesetzt wird.

5. Verfahren nach einem der Ansprüche 1 bis 4, **dadurch gekennzeichnet, dass** die Umsetzung in der zweiten Stufe bei einer Temperatur von 50 bis 90 °C durchgeführt wird.

## Claims

1. Process for preparing robenidine and its derivatives of the general formula 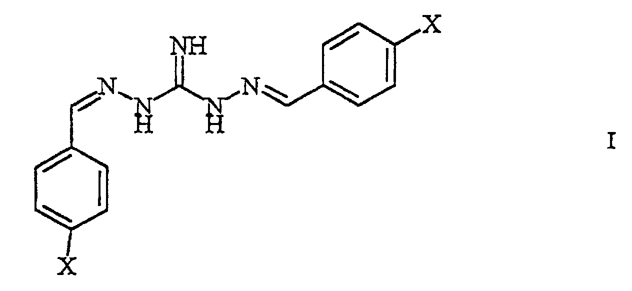 in which X is a halogen atom, and salts thereof, **characterized in that** in a first step hydrazine hydrate is reacted with a cyano compound of the general formula
Y-CN II
in which Y is a halogen atom or tosyl, in a C₁₋₄ alcohol or a C₁₋₄ alcohol-water mixture as solvent to give a diaminoguanidine of the general formula 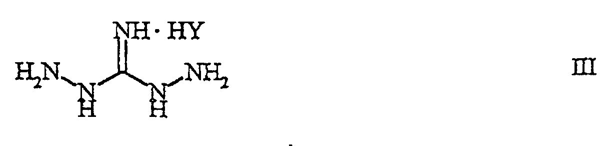 in which Y is as defined above, and this, without isolation, is converted directly in a second step with a p-halobenzaldehyde of the general formula 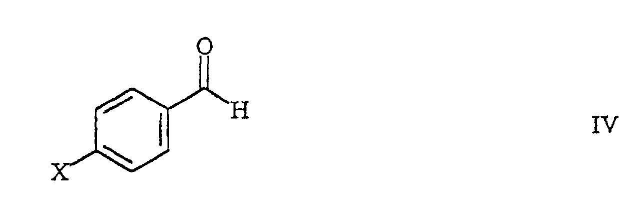 in which X is as defined above, in a C₁₋₄-alcohol-water mixture as solvent into the end product of the formula I.

2. Process according to Claim 1, **characterized in that** the reaction in the first step is carried out at a temperature of from -30 to 50°C.

3. Process according to either of Claims 1 and 2, **characterized in that** the cyano compound used is cyanogen chloride.

4. Process according to one of Claims 1 to 3, **characterized in that** the p-halobenzaldehyde used in the second step is p-chlorobenzaldehyde.

5. Process according to one of Claims 1 to 4, **characterized in that** the reaction in the second step is carried out at a temperature of from 50 to 90°C.

## Revendications

1. Procédé de fabrication de la robénidine et de ses dérivés de formule générale 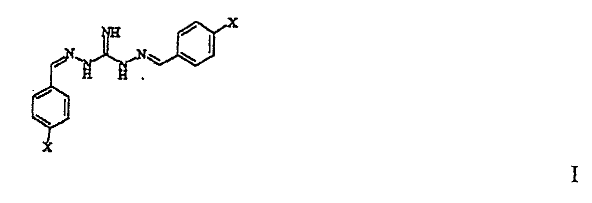 dans laquelle X représente un atome d'halogène, ainsi que de sels, **caractérisé par le fait que** dans une première étape, on transforme l'hydrate d'hydrazine avec un composé de cyanure de formule générale
Y-CN II
dans laquelle Y représente un atome d'halogène ou tosyle, au sein d'un alcool en C₁₋₄ ou d'un mélange d'alcool en C₁₋₄ et d'eau en tant que solvant, en diaminoguanidine de formule générale 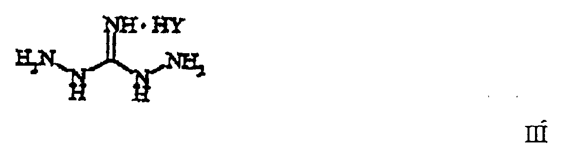 dans laquelle Y a la signification indiquée et qu'au cours d'une deuxième étape, on transforme cette diaminoguanidine, sans isolation, directement avec un p-halogènbenzaldéhyde de formule générale 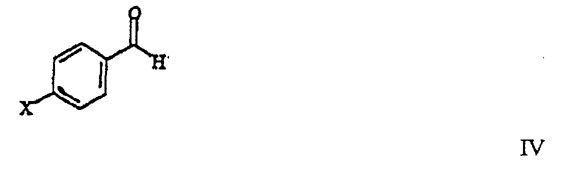 dans laquelle X a la signification indiquée, au sein d'un mélange eau-alcool en C₁₋₄, en tant que solvant dans le produit final de formule I.

2. Procédé selon la revendication I **caractérisé par le fait qu'**au cours de la première étape, la transformation s'effectue à une température de -30 à 50 °C.

3. Procédé selon l'une des revendications 1 ou 2, **caractérisé par le fait que** le composé cyanure utilisé est du cyanure de chlore.

4. Procédé selon l'une des revendications 1 à 3, **caractérisé par le fait que**, dans la deuxième étape, le p-halogènbenzaldéhyde utilisé est le p-chlorobenzaldéhyde.

5. Procédé selon l'une des revendications 1 à 4, **caractérisé par le fait que** la transformation au cours de la deuxième étape s'effectue à une température de 50 à 90 °C.
